# EUROPEAN PATENT APPLICATION

(11) **EP 4 589 296 A1**
(43) Date of publication of application: **23.07.2025**
(21) Application number: 25151898.1
(22) Date of filing: 15.01.2025
(51) Int. Cl.: G01N 33/24, G01N 27/02, A01G 25/16

(54) **SENSOR SYSTEM AND METHOD FOR EVALUATING A STATE OF AN ENVIRONMENT INCLUDING A SOIL**

(30) Priority: 17.01.2024 EP 24152415
(71) Applicant: FYTA GmbH, 10777 Berlin (DE)
(72) Inventor: Nassif, Claudia, 10787 Berlin (DE); Rigalli, Nicolas Francisco, 10551 Berlin (DE); Devagekar, Somesh Shivraj, 13599 Berlin (DE)
(74) Representative: Patentanwaltskanzlei Hinkelmann

(57) **Abstract**

The present invention relates to a sensor system for evaluating a state of an environment including a soil 11 and associated to an organism 12 for living by drawing water and nutrients from the soil 11, the sensor system comprising: a probe 1 insertable into the soil 11 for performing measurements of impedances of the soil 11 at the probe 1, a voltage supply circuit 13 for subjecting the soil 11 to at least two electric signals by the probe 1, each electric signal having a respective predetermined amplitude and a respective frequency between 100 kHz and 5 MHz, the respective frequencies of the at least two electric signals being mutually different, a sensing circuit 4 for obtaining respective output signals from the soil 11 subjected to the electric signals and indicative of a respective impedance, including a rectifying circuit 14 for rectifying the output signals to obtain absolute values of the output signal, a temperature sensor 5 associated to the sensing circuit 4 for performing temperature measurements at the soil 11 to obtain a temperature measured upon subjecting the soil 11 to the electric signals, a communication circuit 7 for communicating the absolute values and the temperature thus obtained, and a forwarding device 8 configured for receiving data from the communication circuit 7 including the absolute values and the temperature. Further, the forwarding device 8 is configured to store secular data of the soil 11 and the organism 12 including a specification of a dry composition of the soil 11 and a specification of the organism 12, and configured for forwarding the secular data with the absolute values and the temperature with the received data, and the sensor system comprises an evaluation device 17 configured for receiving and storing the forwarded data, and secular data, into a time series 19, evaluating the specification of the soil 11 for determining a soil type of the soil 11 from a list of soil types 20 stored in the evaluation device 17, and determining the state of the environment from the time series 19 by comparing the time series 19 with a multiplicity of model time series 21 including model absolute values, model temperatures, model soil types, model organisms, and model states correlated to one another and stored in a database accessible by the evaluation device 17, and determining the state to be equal to a model state in the database model included in one of the multiplicity of model time series 21 closest to the time series 19. The invention also pertains to a related method for evaluating a state of an environment including a soil 11 and associated to an organism 12 for living by drawing water and nutrients from the soil 11.

## Description

The present invention relates to a sensor system for evaluating a state of an environment including a soil and associated to an organism for living by drawing water and nutrients from the soil, the sensor system comprising: A probe insertable into the soil for performing measurements of impedances of the soil at the probe; a voltage supply circuit for subjecting the soil to at least two electric signals by the probe, each electric signal having a respective predetermined amplitude and a respective frequency between 100 kHz and 5 MHz, the respective frequencies of the at least two electric signals being mutually different, a sensing circuit for obtaining respective output signals from the soil subjected to the electric signals and indicative of a respective impedance, including a rectifying circuit for rectifying the output signals to obtain absolute values of the output signal, a temperature sensor associated to the sensing circuit for performing temperature measurements at the organism to obtain a temperature measured upon subjecting the soil to the electric signals, a communication circuit for communicating the absolute values and the temperature thus obtained, and a forwarding device configured for receiving data from the communication circuit including the absolute values and the temperature.

The present invention also relates to a method for evaluating a state of an environment including a soil and associated to an organism for living by drawing water and nutrients from the soil, comprising: Performing measurements of impedances of the soil by subjecting the soil to at least two electric signals, each electric signal having a respective predetermined amplitude and a respective frequency between 100 kHz and 5 MHz, the respective frequencies of the at least two electric signals being mutually different, obtaining respective output signals from the soil subjected to the electric signals and indicative of a respective impedance, rectifying the output signals to obtain absolute values of the output signal, performing a temperature measurement at the organism to obtain a temperature measured upon subjecting the soil to the electric signals, communicating the absolute values and the temperature thus obtained, and receiving data from the communication circuit including the absolute values and the temperature by a forwarding .

The article entitled "A Novel Low-Cost Instrumentation System for Measuring the Water Content and Apparent Electrical Conductivity of Soils" and published by A. K. Rego Segundo et al., Sensors 15 (2015) 25546, discloses a sensor device and a method according to the respective generic type as defined above. Based on considerations of scarcity of drinking water, the article introduces instrumentation including a sensor prototype for measuring water content and electrical conductivity of a soil. Experimental results are shown to demonstrate accuracy of evaluation models offered to evaluate water content and conductivity from signals obtained from the sensor prototype, and further development potential to be evaluated in testing the sensor prototype with more soils. The soils under consideration in this article are soils whereon agriculture for producing crops is exercised.

The article entitled "Review of Research Progress on Soil Moisture Sensor Technology" and published by L. Yu et al, Int. J. Agric. & Biol. Eng. 14,4 (2021) 32, provides an overview over sensor technology applicable for measuring soil moisture. Again, the soils under consideration are soils whereon agriculture for producing crops is practised. Problems with known sensors and highlighted in the article include accuracy and applicability for various soils, and costs of sensors for application in agriculture.

Besides agriculture as dedicated to producing crops for human and animal feeding and support, gardening in the context of human life and work, such as in a private household, eventually involving production of some crop at a private household or within an office surrounding and generally involving keeping plants and other organisms living in and feeding from amounts of soil for primarily aesthetic purposes is also an activity pursued worldwide. Such gardening is usually performed with low support by technology except use of small traditional tools. Yet, gardening also requires related knowledge in keeping and growing plants with watering, fertilizing, and exposing to a properly temperate and lighted environment. Given the advantages of having plants in a private household or an office, there is a need for support in the care for such plants to motivate creation and maintenance of gardens in a household or an office regardless of respectively available competence for plant care, considering that even plants are complex living organisms with specific requirements for their support, and related requirements for diagnosing states and necessities of such plants and the environments including soils that they are living upon and feeding from.

Accordingly, there is a need for means and methods for properly diagnosing environments of plants in keeping plants not only in agriculture and also in household and office.

Therefore, it is an object of the present invention to provide a sensor system and a method for evaluating a state of an environment including a soil and associated to an organism for growing by drawing water and nutrients from the soil which is suitable for application in a household or office.

The present invention provides solutions to such object and other objects with the sensor system and the method according to the respective independent claim attached. Preferred embodiments of the sensor system and the method, respectively, are defined in dependent claims as well as in subsequent disclosure, and may be applied in combinations insofar as not excluded for technical reasons. Preferred embodiments of the sensor system correspond to preferred embodiments of the method and vice versa, even if not explicitly stated herein.

Accordingly, there is provided, in accordance with the invention, a sensor system for evaluating a state of an environment including a soil and associated to an organism for living by drawing water and nutrients from the soil, the sensor system comprising: A probe insertable into the soil for performing measurements of impedances of the soil at the probe; a voltage supply circuit for subjecting the soil to at least two electric signals by the probe, each electric signal having a respective predetermined amplitude and a respective frequency between 100 kHz and 5 MHz, the respective frequencies of the at least two electric signals being mutually different, a sensing circuit for obtaining respective output signals from the soil subjected to the electric signals and indicative of a respective impedance, including a rectifying circuit 14 for rectifying the output signals to obtain absolute values of the output signal, a temperature sensor associated to the sensing circuit for performing temperature measurements at the organism to obtain a temperature measured upon subjecting the soil to the electric signals, a communication circuit for communicating the absolute values and the temperature thus obtained, and a forwarding device configured for receiving data from the communication circuit including the absolute values and the temperature. Further, the forwarding device is configured to store secular data of the soil and the organism including a specification of a dry composition of the soil and a specification of the organism, and configured for forwarding the secular data with the absolute values and the temperature with the received data, and the sensor system comprises an evaluation device configured for receiving and storing the forwarded data, and secular data, into a time series, evaluating the specification of the soil for determining a soil type of the soil from a list of soil types stored in the evaluation device, and determining the state of the environment from the time series by comparing the time series with a multiplicity of model time series including model absolute values, model temperatures, model soil types, model organisms, and model states correlated to one another and stored in a database accessible by the evaluation device, and determining the state to be equal to a model state in the database model included in one of the multiplicity of model time series closest to the time series.

In addition, there is provided, in accordance with the invention, a method for evaluating a state of an environment including a soil and associated to an organism for living by drawing water and nutrients from the soil, comprising: Performing measurements of impedances of the soil by subjecting the soil to at least two electric signals, each electric signal having a respective predetermined amplitude and a respective frequency between 100 kHz and 5 MHz, the respective frequencies of the at least two electric signals being mutually different, obtaining respective output signals from the soil subjected to the electric signals and indicative of a respective impedance, rectifying the output signals to obtain absolute values of the output signal, performing a temperature measurement at the organism to obtain a temperature measured upon subjecting the soil to the electric signals, communicating the absolute values and the temperature thus obtained, and receiving data from the communication circuit including the absolute values and the temperature by a forwarding device. Further, the forwarding device stores secular data of the soil and the organism including a specification of a dry composition of the soil and a specification of the organism and forwards the secular data with the absolute values and the temperature with the received data, and an evaluation device receives and stores the forwarded data, and secular data, into a time series, evaluates the specification of the soil for determining a soil type of the soil from a list of soil types stored in the evaluation device, and determines the state of the soil from the time series by comparing the time series with a multiplicity of model time series including model absolute values, model temperatures, model soil types, model organisms, and model states correlated to one another and stored in a database accessible by the evaluation device, and determines the state to be equal to a model state in the database model included in one of the multiplicity of model time series closest to the time series.

In accordance with the present invention, data pertaining to an environment including a soil and associated to an organism living by drawing water and nutrients from the soil, such data including absolute values of impedances of the soil and temperatures at the organism are collected by a sensing device and a temperature sensor and forwarded to a forwarding device which forwards the absolute values and the temperatures with secular data of the soil and the organism.

In accordance with the invention, the secular data of the soil and the organism include data which are stationary or quasi-stationary in time by varying much more slowly in relation to the absolute values and the temperatures and serve to characterize the soil and the organism. They may include a specification of the dry composition of the soil, taxonomic information pertaining to the organism, data of a receptacle such as a pot which contains the soil and the organism, and pictorial representations of the organism and the soil. Identification data of the probe and the forwarding device as well as data necessary for securing transmission to the evaluation device may also be included in the secular data. Secular data received by the evaluation device and pertaining to the organism and the soil are stored in a time series with a specification of the soil, and the state of the environment is determined from the time series by comparing the time series with a multiplicity of model time series including model absolute values, model temperatures, model soil types, model organisms, and model states correlated to one another and stored in a database accessible by the evaluation device, to be equal to a model state in the database model included in one of the multiplicity of model time series closest to the time series.

Such determination may be performed by a self-learning system such as a trained neural network, training to be performed with model time series obtained experimentally or designed in appropriation of results of related experiments, the model time series including model states of the soil correlated with model absolute values and temperatures. Consideration of further data in determining a state of the soil such as data specifying the organism's exposure to light may be included.

Types of soil are primarily considered to be preparations of commonly and commercially available soils which may be composed of matters such as clay, silt, limestone, pumice, peat, composted matter, sand, coir, sphagnum moss, bark, bentonite, zeolites, gravel and perlite or obsidian. Such soils may be provided for general use, or for use with specific plants such as cactuses, orchids, or plants originated from bogs and requiring soils of high acidity.

In considering absolute values of impedances and temperatures only and evaluating time series of these, the present invention provides a reliable state of the environment including a soil and associated to an organism including a specification of the soil's humidity, as values which are determinative in everyday care for an organism living upon and feeding from a soil. The invention allows to obtain status with high precision by relying on time series of measurements instead of single measurements as in the prior art and avoids complex evaluation schemes to obtain concrete values of humidity and fertility from single impedance measurements by relying on comparison of time series with model time series. Thereby, the invention supports provision of instructions to a user for properly watering the soil with the organism. In addition, the invention enables an evaluation of the soil's nutrient content as another constituent of the state of the soil, providing guidance to a user for properly fertilizing the soil with the organism. Thereby, the invention enables proper care for an organism living on and feeding from a soil by users not having professional skills in plant care at their disposal.

The forwarding device applied in the invention may be any mobile device for use by a user such as a smartphone or a portable computer such as a tablet and properly programmed by a dedicated application program to be configured for connecting to the evaluation device through a mobile communication network and via Internet or Local Area Network. The forwarding device may also be any other device, be it standalone or included in any other device included in the sensor system and properly connected to a communication network.

The voltage supply circuit and rectifying circuit may be provided according to common knowledge in electronics, for example as a variable-frequency oscillator or two selectable oscillators with precisely determined amplitude, and as precision rectifier, respectively, in analog or digital circuitry or a combination of these, applying operational amplifiers, for example. The precision rectifier if provided in analog circuitry may be coupled to an analog-digital-converter for providing digitalized measurement results to the communication circuit, the forwarding device, and the evaluation device.

Preferably, the frequencies for measuring impedances are between 100kHz and 5 MHz, and more preferred between 100 kHz and 2 MHz.

Preferably, the probe includes two electrodes and a housing, the electrodes protruding from the housing at a distance between each other, and inserted into the soil, with the housing touching the soil. More preferred, each electrode is a cylinder having an axial length between 5 cm and 35 cm, and a radial diameter between 3 mm and 8 mm.

In accordance with a preferred embodiment of the invention, the evaluation device is configured for sending the state of the environment to the forwarding device, and the forwarding device is configured for receiving, storing, and displaying the state of the environment. Thereby, a user may obtain information about the state of the environment from or via the forwarding device and be alerted to provide or adjust care given to organism such as watering, fertilizing, or reducing excess watering. The precision attainable by applying the invention may even allow to specify measures to be taken quantitatively, such as specifying an amount of water or an amount of fertilizer to be provided into the soil.

In accordance with a preferred embodiment of the invention, each state of the environment and each model state includes a respective first humidity value specifying an actual humidity of the soil, and a respective second humidity value specifying a target humidity of the soil. Thereby, information related to properly watering the soil and the organism is provided for consideration by a user to improve care for the organism.

In accordance with another preferred embodiment of the invention, each state of the environment and each model state includes a respective first fertility value specifying an actual nutrient content of the soil, and a second fertility value specifying a target nutrient content of the soil. Thereby, information related to properly fertilizing the soil and the organism is provided for consideration by a user to improve care for the organism.

In accordance with a further preferred embodiment of the invention, the probe includes two electrodes and a housing, the electrodes protruding from the housing at a distance between each other and configured to be inserted into the soil with the housing touching the soil. Thereby, measurements of conductivity in the soil are facilitated.

In accordance with an additional preferred embodiment of the invention, the impedance sensor is configured for performing a first measurement wherein the at least two electric signals comprise a first signal which is a square signal having a frequency of about 100 kHz and a second signal which is a square signal having a frequency of about 1 MHz. Frequencies within the range specified may be handled properly and easily by currently available electronics, avoiding adverse effects on electronics neighboured to the sensing device, and provide reliable and reproducible measurements.

In accordance with yet another preferred embodiment of the invention, a low-pass filter is provided between the sensing circuit and rectifying circuit for filtering each output signal prior to being forwarded to rectifying circuit. More preferred, the low-pass filter is a passive filter. The low-pass filter also assists in handling the conductivity measurements properly and easily, avoiding adverse effects from electronics neighboured to the sensing device, and assist in providing reliable and reproducible measurements.

In accordance with yet a further preferred embodiment of the invention, a clock is associated to the probe, the impedance sensor and the temperature sensor are configured to obtain measurements at predetermined times to be specified by the clock, and the communication circuit is configured to forward an associated measurement time together with each absolute value and each temperature measured to the forwarding device. More preferred, the probe is configured to repeat each measurement after expiry of a predetermined interval of time. Even more preferred, the predetermined interval is about one hour. This assists in providing the time series for evaluating the state of the environment with measured values in a regular sequence which improves reliability of evaluation.

In accordance with yet an additional preferred embodiment of the invention, the probe is configured to measure a sample of a soil with a known state and forward the obtained absolute value to the evaluation device for storing data obtained from the sample in a model time series in the database. This assists in obtaining model time series for calibrating or training the evaluation device and the database that it includes and supports an evaluation device structured as a self-learning device.

In accordance with still another preferred embodiment of the invention, the forwarding device is configured to obtain the secular data by prompting a user applying the soil for the organism to live from to enter the secular data into the forwarding device.

In accordance with still a further preferred embodiment of the invention, the sensing circuit includes an operation amplifier connected as an inverting amplifier, with the electrodes connected to form an input impedance to the inverting amplifier.

In accordance with again a further preferred embodiment of the invention, rectifying circuit includes an operation amplifier connected as a precision rectifier.

In accordance with a concomitant preferred embodiment of the invention, the at least two electric signals comprise a first signal which is a square signal having a frequency of about 100 kHz and a second signal which is a square signal having a frequency of about 1 MHz.

Preferably, the organism that the soil is associated to is a plant, particularly a plant suited for growing in a subtropical or tropical climate, a pot plant, or an indoor plant.

The invention is open for application in a private surrounding such as a household or an office as well as for application in a professional surrounding such as agriculture and related business. In any application, the invention assists in keeping organisms growing upon and feeding from soils properly watered and properly fertilized, to assist in optimizing their environments for best growth and best health of such organisms.

Subsequently, preferred embodiments of the invention with related advantages are discussed in detail with reference to the accompanying drawing. The drawing figures s are schematic and not implied or intended to exhibit embodiments of components properly scaled.

In particular,
- Fig. 1: exhibits a schematic representation of a probe for application with the invention;
- Fig. 2: exhibits a forwarding device configured for communication with a probe;
- Fig. 3: exhibits an organism growing upon soil in a pot with an associated probe;
- Fig. 4: exhibits the circuitry in Fig. 1's probe in more detail;
- Fig. 5: exhibits an evaluation device for application with the invention schematically;
- Fig. 6: exhibits measured values obtained in a practical implementation of the invention;
- Fig. 7: exhibits a time series of an impedance measured in a soil by a probe; and
- Fig. 8: exhibits time series of a light condition measured at a soil by a probe.

The Figures exhibit preferred embodiments of a sensor system for evaluating a state of an environment including a soil 11 and associated to an organism 12 for living by drawing water and nutrients from soil 11.

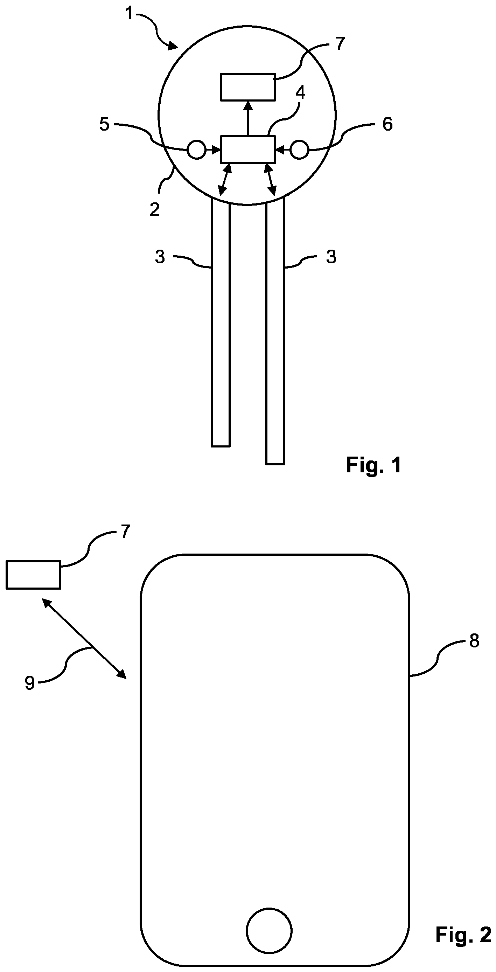

As shown in Figs. 1, 3, and 4, the sensor system comprises a probe 1 including a housing 2 and two electrodes 3, the latter protruding from housing 2 at a distance between each other and configured to be inserted into soil 11 with housing 2 touching soil 11 for performing measurements of impedances of soil 11 at probe 1, each electrode 3 being a cylinder having an axial length between 5 cm and 35 cm, and a radial diameter between 3 mm and 8 mm.

A voltage supply circuit 13 is provided for subjecting soil 11 to at least two electric signals by probe 1, each electric signal having a respective predetermined amplitude and a respective frequency between 100 kHz and 5 MHz, the respective frequencies of the at least two electric signals being mutually different. Preferably, sensing circuit 4 is configured for performing a first measurement wherein the at least two electric signals comprise a first signal which is a square signal having a frequency of about 100 kHz and a second signal which is a square signal having a frequency of about 1 MHz.

Probe 1 includes a sensing circuit 4 for obtaining respective output signals from soil 11 subjected to the electric signals and indicative of a respective impedance, including a rectifying circuit 14 for rectifying the output signals to obtain absolute values of the output signal, and a temperature sensor 5 associated to sensing circuit 4 for performing temperature measurements at soil 11 to obtain a temperature measured upon, in particular concurrently with, subjecting soil 11 to the electric signals. A low-pass filter, specifically a passive low-pass filter, may be provided between electrodes 3 and rectifying circuit 14. Sensing circuit 4 includes an operation amplifier connected as an inverting amplifier, with the electrodes 3 connected to form an input impedance to the inverting amplifier. Rectifying circuit 14 includes an operation amplifier connected as a precision rectifier and an analog-digital-converter configured for converting the absolute value obtained into a digital value.

A light sensor 6 for measuring exposure to environmental light is also associated to sensing circuit 4.

Communication circuit 7 is provided in probe 1 for communicating the absolute values and the temperature thus obtained, and a forwarding device 8 as exhibited in Fig. 2 is provided and configured for receiving data from communication circuit 7 including the absolute values and the temperature, as indicated by bidirectional arrow 9. Specifically, forwarding device 8 is a mobile device 8 for general use and communication and properly programmed by a dedicated application program such as a smartphone or a portable computer. Forwarding device 8 is configured to store secular data of soil 11 and organism 12 including a specification of a dry composition of soil 11 and a specification of organism 12, and configured for forwarding the secular data with the absolute values and the temperature with the received data. The secular data may include further data as specified hereinabove.

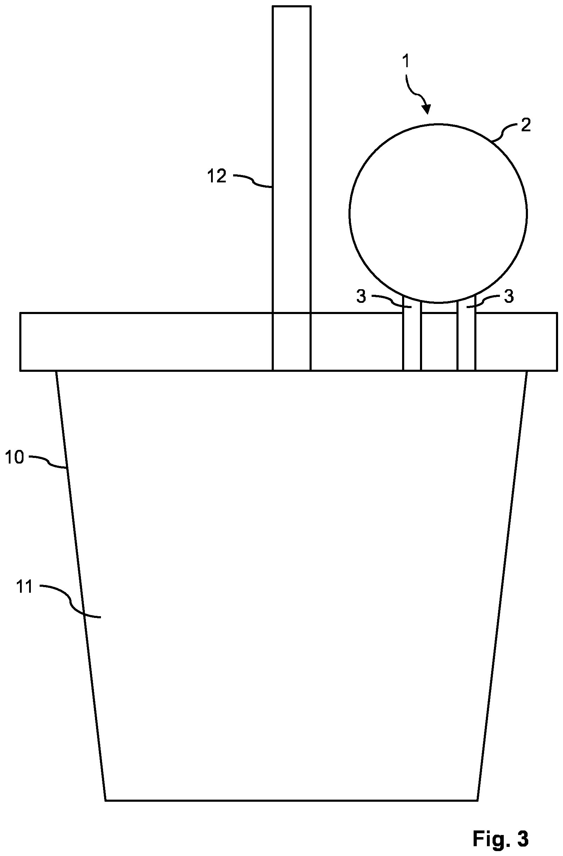

Fig. 3 shows a pot 10 and soil 11 contained in pot 10, and organism 12 symbolized by a stalk growing upon and feeding from soil 11. Probe 1 with housing 2 almost touching soil 11 is also shown, together with electrodes 3 that should be fully inserted into soil 11 for proper operation.

Organism 12 is a plant, particularly a plant suited for growing in a subtropical or tropical climate, a pot plant, or an indoor plant.

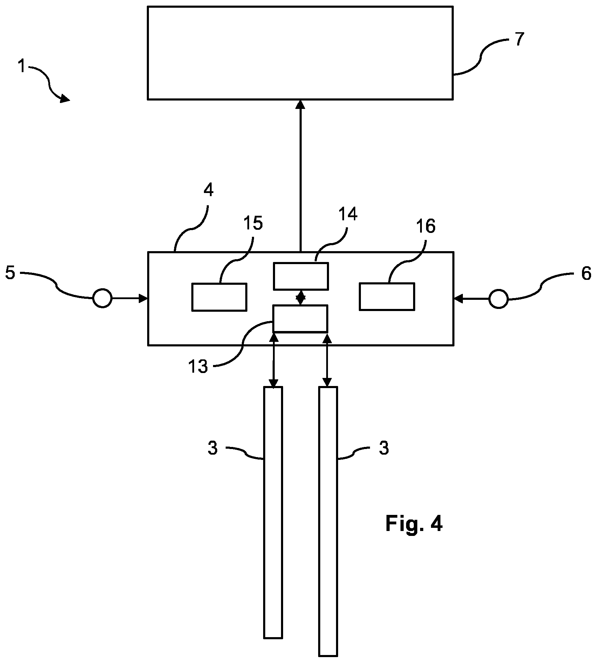

As exhibited in Fig. 4, probe 1 includes, besides sensing circuit 4, a voltage supplying circuit 13 such as an oscillator for delivering the electric signals with precisely set amplitudes, a clock 15 and a probe memory 16. With clock 15 associated to probe 1, sensing circuit 4, temperature sensor 5, and light sensor 6, are configured to obtain measurements at predetermined times to be specified by clock 15, and communication circuit 7 is configured to forward an associated measurement time together with each absolute value and each temperature measured to forwarding device 8. Thus, probe 1 is configured to repeat each measurement after expiry of a predetermined interval of time, which interval of time may be one hour. Probe memory 16 serves specifically to store results of measurements prior to communicating them to forwarding device 8.

Forwarding device 8 is also configured to obtain the secular data by prompting a user applying soil 11 for organism 12 to live from to enter the secular data into forwarding device 8.

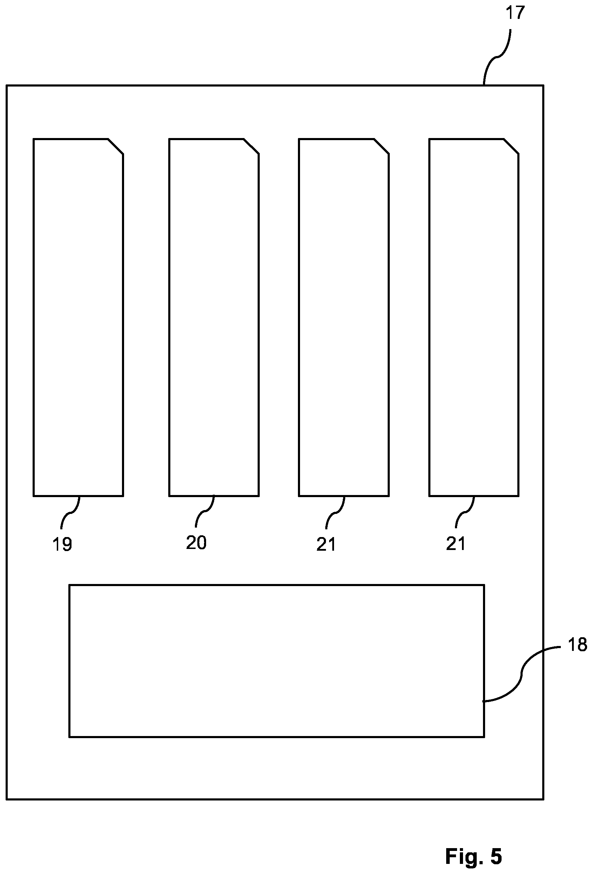

Fig. 5 exhibits an evaluation device 17 included in the sensor system and configured for receiving and storing the forwarded data, and secular data, into a time series 19, evaluating the specification of soil 11 for determining a soil type of soil 11 from a list of soil types 20 stored in evaluation device 17, and determining the state of the soil 11 from time series 19 by comparing time series 19 with a multiplicity of model time series 21 including model absolute values, model temperatures, model soil types, model organisms, and model states correlated to one another and stored in a database accessible by the evaluation device 17, and determining the state to be equal to a model state in the database model included in one of the multiplicity of model time series 21 closest to the time series 19.

Evaluation device 17 is configured for sending the state of the soil to forwarding device 8 after determination, and forwarding device 8 is configured for receiving, storing, and displaying the state of the soil. Thereby, a user may obtain information about the state of the soil from forwarding device 8 and be alerted to provide or adjust care given to organism 12 and its proper environment such as watering, fertilizing, or reducing excess watering. The precision attainable by applying the invention even allows to specify measures to be taken quantitatively, such as specifying an amount of water or an amount of fertilizer to be provided into soil 11. Further, forwarding device 8 may communicate a state of the soil just received to probe 1, and probe 1 may include another display device such as a light-emitting diode and applicable for assisting in alerting a user to provide or adjust care for organism 12 and its proper environment.

Each state of soil 11 and each model state includes a respective first humidity value specifying an actual humidity of soil 11, and a respective second humidity value specifying a target humidity of soil 11.

Further, each state of soil 11 and each model state includes a respective first fertility value specifying an actual nutrient content of soil 11, and a second fertility value specifying a target nutrient content of soil 11.

Probe 1 is also configured to measure a sample of a soil 11 with a known state and forward the obtained absolute value to the evaluation device 17 for storing data obtained from the sample in a model time series 21 in the database.

A method for evaluating a state of an environment including a soil 11 and associated to an organism 12 for living by drawing water and nutrients from the soil 11 may be implemented by applying the components described above in procedures as described above.

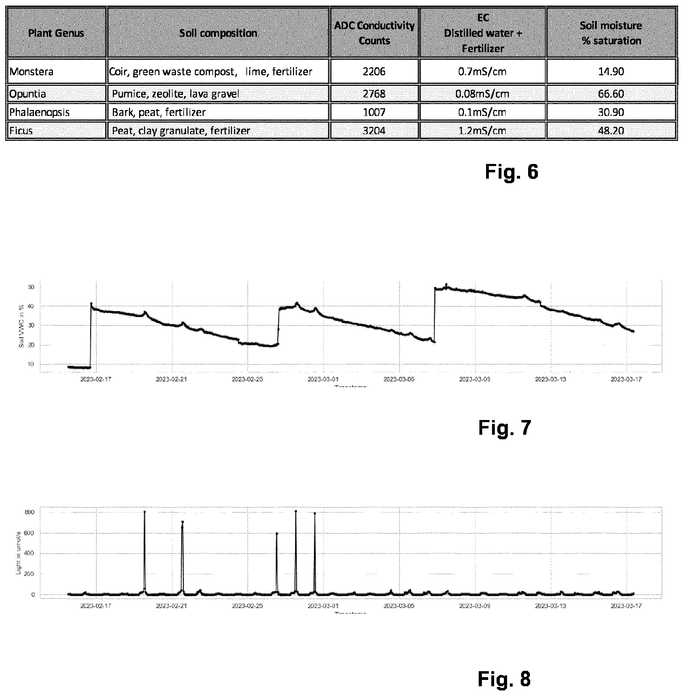

Fig. 6 exhibits a table of plant genus types with compositions of commercially available soils and related specific conductivities as occurring with specified humidity or moisture, moisture specified as relative saturation with 100% specifying the maximum amount that the soil will hold without dripping. Such values have been obtained with a probe 1 as explained in detail hereinabove and are to be expected in measurements of impedances according to the invention.

Figs. 7 and 8 exhibit time series of impedance measurements in Fig. 7 and correlated light intensity measurements in Fig. 8. The steps in Fig. 7 exhibit effects of watering the plant with subsequent loss of humidity from the soil by evaporation from the soil itself and from the organism feeding from the soil. The generally decreasing flanks in impedance measurements also reflect events of extraordinarily high exposure to light, resulting in small but well noticeable spikes in the flanks which exhibit an organism's reaction to increased exposure to light. This highlights the fact that time series allow a significant increase in precision of conclusions about the state of a soil which is applied by an organism such as a plant feeding from it.

In considering absolute values of impedances and temperatures and evaluating time series of these, the present invention provides a reliable state of the organism's environment including the soil, the state including a specification of the soil's humidity as values which are determinative in everyday care for an organism living upon and feeding from the soil. The invention allows to obtain status with high precision by relying on time series of measurements and avoids complex evaluation schemes to obtain concrete values of humidity and fertility from single impedance measurements by relying on comparison of time series with model time series. Thereby, the invention supports provision of instructions to a user for properly watering the soil with the organism. In addition, the invention enables an evaluation of the soil's nutrient content as another constituent of the state of the environment, providing guidance to a user for properly fertilizing the soil. Thereby, the invention enables proper care for an organism living on and feeding from a soil by users not having professional skills in plant care at their disposal.

### LIST OF REFERENCE NUMERALS

1. Probe
2. Housing
3. Electrode
4. Sensing circuit
5. Temperature sensor
6. Light sensor
7. Communication circuit
8. Forwarding Device, Mobile Device
9. Communication link
10. Pot
11. Soil
12. Organism
13. Voltage supply circuit
14. Rectifying circuit
15. Clock
16. Probe memory
17. Evaluation device
18. Processor
19. Time series
20. List of soil types
21. Model time series

## Claims

1. A sensor system for evaluating a state of an environment including a soil (11) and associated to an organism (12) for living by drawing water and nutrients from the soil (11), the sensor system comprising:
- a probe (1) insertable into the soil (11) for performing measurements of impedances of the soil (11) at the probe (1);
- a voltage supply circuit (13) for subjecting the soil (11) to at least two electric signals by the probe (1), each electric signal having a respective predetermined amplitude and a respective frequency between 100 kHz and 5 MHz, the respective frequencies of the at least two electric signals being mutually different;
- a sensing circuit (4) for obtaining respective output signals from the soil (11) subjected to the electric signals and indicative of a respective impedance, including a rectifying circuit (14) for rectifying the output signals to obtain absolute values of the output signal;
- a temperature sensor (5) associated to the sensing circuit (4) for performing temperature measurements at the organism (12) to obtain a temperature measured upon subjecting the soil (11) to the electric signals;
- a communication circuit (7) for communicating the absolute values and the temperature thus obtained; and
- a forwarding device (8) configured for receiving data from the communication circuit (7) including the absolute values and the temperature; **characterized in that**
the forwarding device (8) is configured to store secular data of the soil (11) and the organism (12) including a specification of a dry composition of the soil (11) and a specification of the organism (12), and configured for forwarding the secular data with the absolute values and the temperature with the received data; and
the sensor system comprises an evaluation device (17) configured for receiving and storing the forwarded data and secular data into a time series (19), evaluating the specification of the soil (11) for determining a soil type of the soil (11) from a list of soil types (20) stored in the evaluation device (17), and determining the state of the environment from the time series (19) by comparing the time series (19) with a multiplicity of model time series (21) including model absolute values, model temperatures, model soil types, model organisms, and model states correlated to one another and stored in a database accessible by the evaluation device (17), and determining the state to be equal to a model state in the database model included in one of the multiplicity of model time series (21) closest to the time series (19).

2. Sensor system according to claim 1, wherein the evaluation device (17) is configured for sending the state of the soil to the forwarding device (8), and the forwarding device (8) is configured for receiving, storing, and displaying the state of the environment.

3. Sensor system according to claim 2, wherein the forwarding device (8) is a mobile device (8).

4. Sensor system according to one of the preceding claims, wherein each state of the environment and each model state includes a respective first humidity value specifying an actual humidity of the soil (11), and a respective second humidity value specifying a target humidity of the soil (11).

5. Sensor system according to one of the preceding claims, wherein each state of the environment and each model state includes a respective first fertility value specifying an actual nutrient content of the soil (11), and a second fertility value specifying a target nutrient content of the soil (11).

6. Sensor system according to one of the preceding claims, wherein the probe (1) includes two electrodes (3) and a housing (2), the electrodes (3) protruding from the housing (2) at a distance between each other and configured to be inserted into the soil (11) with the housing (2) touching the soil.

7. Sensor system according to claim 6, wherein each electrode (3) is a cylinder having an axial length between 5 cm and 35 cm, and a radial diameter between 3 mm and 8 mm.

8. Sensor system according to one of the preceding claims, wherein the sensing circuit (4) is configured for performing a first measurement wherein the at least two electric signals comprise a first signal which is a square signal having a frequency of about 100 kHz and a second signal which is a square signal having a frequency of about 1 MHz.

9. Sensor system according to one of the preceding claims, wherein a low-pass filter is provided in the sensing circuit (4) for filtering each output signal prior to being rectified the rectifying circuit (14).

10. Sensor system according to claim 9, wherein the low-pass filter is a passive filter.

11. Sensor system according to one of the preceding claims, wherein a clock (15) is associated to the probe (1), the sensing circuit (4) and the temperature sensor (5) are configured to obtain measurements at predetermined times to be specified by the clock (15), and the communication circuit (7) is configured to forward an associated measurement time together with each absolute value and each temperature measured to the forwarding device (8).

12. Sensor system according to claim 10, wherein the probe (1) is configured to repeat each measurement after expiry of a predetermined interval of time.

13. Sensor system according to one of the preceding claims, wherein the probe (1) is configured to measure a sample of a soil (11) with a known state and forward the obtained absolute value to the evaluation device (17) for storing data obtained from the sample in a model time series (21) in the database.

14. Sensor system according to one of the preceding claims, wherein the forwarding device (8) is configured to obtain the secular data by prompting a user applying the soil (11) for the organism (12) to live from to enter the secular data into the forwarding device (8).

15. Sensor system according to one of the preceding claims, wherein the sensing circuit (4) includes an operation amplifier connected as an inverting amplifier, with the electrodes (3) connected to form an input impedance to the inverting amplifier.

16. Sensor system according to one of the preceding claims, wherein the rectifying circuit (14) includes an operation amplifier connected as a precision rectifier.

17. Sensor system according to claim 16, wherein the rectifying circuit (14) includes an analog-digital-converter configured for converting the absolute value obtained into a digital value.

18. Sensor system according to one of the preceding claims, wherein the organism (12) is a plant, particularly a plant suited for growing in a subtropical or tropical climate, a pot plant, or an indoor plant.

19. A method for evaluating a state of an environment including a soil (11) and associated to an organism (12) for living by drawing water and nutrients from the soil (11), comprising:
- performing measurements of impedances of the soil (11) by subjecting the soil (11) to at least two electric signals, each electric signal having a respective predetermined amplitude and a respective frequency between 100 kHz and 5 MHz, the respective frequencies of the at least two electric signals being mutually different;
- obtaining respective output signals from the soil (11) subjected to the electric signals and indicative of a respective impedance;
- rectifying the output signals to obtain absolute values of the output signal;
- performing a temperature measurement at the organism (12) to obtain a temperature measured upon subjecting the soil (11) to the electric signals;
- communicating the absolute values and the temperature thus obtained; and
- receiving data from the communication circuit (7) including the absolute values and the temperature by a forwarding device (8); **characterized in that**
the forwarding device (8) stores secular data of the soil (11) and the organism (12) including a specification of a dry composition of the soil (11) and a specification of the organism (12) and forwards the secular data with the absolute values and the temperature with the received data; and
an evaluation device (17) receives and stores the forwarded data, and secular data, into a time series (19), evaluates the specification of the soil (11) for determining a soil type of the soil (11) from a list of soil types (20) stored in the evaluation device (17), and determines the state of the environment from the time series (19) by comparing the time series (19) with a multiplicity of model time series (21) including model absolute values, model temperatures, model soil types, model organisms, and model states correlated to one another and stored in a database accessible by the evaluation device (17), and determines the state to be equal to a model state in the database model included in one of the multiplicity of model time series (21) closest to the time series (19).

20. The method according to claim 19, wherein the state of the environment is sent to the forwarding device (8), and the forwarding device (8) receives, stores, and displays the state of the environment.

21. The method according to one of claims 19 and 20, wherein the at least two electric signals comprise a first signal which is a square signal having a frequency of about 100 kHz and a second signal which is a square signal having a frequency of about 1 MHz.

22. The method according to one of claims 19 to 21, wherein measurements are obtained at predetermined times to be specified by a clock (15), and an associated measurement time is forwarded together with respective absolute values and a respective temperature measured to the evaluation device (17).

23. The method according to one of claims 19 to 22, wherein each measurement is repeated after expiry of a predetermined interval of time.

24. The method according to claim 22, wherein the predetermined interval is about one hour.
